# EUROPEAN PATENT SPECIFICATION

(11) **EP 4 360 086 B1**
(45) Date of publication and mention of the grant of the patent: **07.10.2026**
(21) Application number: 22829486.4
(22) Date of filing: 21.06.2022
(51) Int. Cl.: G10L 25/66, A61B 5/00, G10L 25/30

(54) **SYSTEM AND METHOD OF VOICE BIOMARKER DISCOVERY FOR MEDICAL DIAGNOSIS USING NEURAL NETWORKS**
SYSTEM UND VERFAHREN ZUR ENTDECKUNG VON SPRACHBIOMARKERN FÜR MEDIZINISCHE DIAGNOSEN UNTER VERWENDUNG NEURONALER NETZWERKE
SYSTÈME ET PROCÉDÉ DE DÉCOUVERTE DE BIOMARQUEURS VOCAUX POUR DIAGNOSTIC MÉDICAL EN UTILISANT DES RÉSEAUX NEURONAUX

(30) Priority: 22.06.2021 US 202163213356 P
(43) Date of publication of application: 01.05.2024
(60) Divisional application: 26202371.6
(73) Proprietor: AIVA Global, Inc., Boothwyn, PA 19061 (US)
(72) Inventor: SINGH, Rita, Pittsburgh, Pennsylvania 15217 (US)
(74) Representative: K&L Gates LLP
(86) International application number: PCT/US2022/073052
(87) International publication number: WO 2022/272241

(56) References cited:
- WO-A1-2021/081418
- US-A1- 2012 265 024
- US-A1- 2017 270 919
- US-A1- 2020 029 084
- US-B1- 10 229 672
- US-B2- 8 731 839
- PARVANEH JANBAKHSHI ET AL: "Supervised Speech Representation Learning for Parkinson's Disease Classification", ARXIV.ORG, CORNELL UNIVERSITY LIBRARY, 201 OLIN LIBRARY CORNELL UNIVERSITY ITHACA, NY 14853, 1 June 2021 (2021-06-01), XP081982408
- TABAN ESLAMI ET AL: "ASD-DiagNet: A hybrid learning approach for detection of Autism Spectrum Disorder using fMRI data", ARXIV.ORG, CORNELL UNIVERSITY LIBRARY, 201 OLIN LIBRARY CORNELL UNIVERSITY ITHACA, NY 14853, 16 April 2019 (2019-04-16), XP081169924
- SHAKERI MAHSA ET AL: "Deep Spectral-Based Shape Features for Alzheimer's Disease Classification", 11 December 2016, SAT 2015 18TH INTERNATIONAL CONFERENCE, AUSTIN, TX, USA, SEPTEMBER 24-27, 2015; [LECTURE NOTES IN COMPUTER SCIENCE; LECT.NOTES COMPUTER], SPRINGER, BERLIN, HEIDELBERG, PAGE(S) 15 - 24, ISBN: 978-3-540-74549-5, XP047365079

## Description

### PRIORITY CLAIM

The present application claims priority to United States provisional application Serial No. 63/213,356, filed June 22, 2021, titled "System and Method of Voice Biomarker Discovery for Medical Diagnosis Using Neural Networks."

### BACKGROUND

A biomarker for a medical condition is an objectively specifiable vector or tensor that corresponds to a pattern in one or more multidimensional mathematical spaces (which may or may not be human interpretable or viewable in their entirety), such that it is highly discriminative for that condition in any computational setting. Here the term "medical condition" includes, but is not limited to factors and parameters that are relevant to the human body and its correct functioning, such as diseases, syndromes, infections, physical and physiological abnormalities, etc. Voice is known to carry biomarkers for multiple medical conditions, but it is hard to measure them objectively even for those conditions for which biomarkers have been observed to exist. For other conditions that have biological pathways to the human vocal production mechanism, biomarkers can be hypothesized to be present in voice, even if they are not human-observable (i.e. they may be imperceptible).

A number of biomarkers and signal features related to biomarkers have already been identified in the scientific literature. These include, but are not limited to spectra, spectrographic representations, voicing-onset time, formants, formant bandwidth, modulation, harmonicity, fundamental frequency and its harmonics, jitter, shimmer, resonances and antiresonances, etc. These features, which may be directly derived from the raw signal, spectrographic time-frequency representations and other transform domains, are derived using various mathematically well-motivated digital signal processing (DSP) or other Machine Learned Signal Processing (MLSP) techniques, and may be viewed as the "measurable" properties of the voice signal. However, the set of such measurements is limited and enumerable, due to the limited number of digital signal processing (DSP) algorithms available to compute them, and by the time-frequency (and other) resolution tradeoffs implicit in them, and may not be sufficiently diverse, or of fine-enough resolution to capture all biomarkers relevant to a target medical condition.

Alternate approaches use neural networks to derive features from the incoming signal, to classify a medical condition. While this approach requires less expert knowledge of the target medical condition or its influence on the voice signal, it derives abstract, uninterpretable features, which may in turn lose information about the measurable properties of the signal that can be effectively derived by the more conventional signal-processing approach described earlier. In the absence of sufficiently large, diverse training data, it also remains uncertain whether these methods derive actual biomarkers for the target condition, or merely some incidental features that are specific to the training data or conditions used.

WO 2021/081418 A1 discloses a method for detecting behavioral or mental health conditions in a subject with an acoustic model. The model comprises an encoder and a classifier. The method comprises obtaining a speech sample comprising a plurality of speech segments from the subject, processing the speech sample with the encoder to generate an abstract feature representation of the speech sample, and processing the abstract feature representation with the classifier to generate an output indicative of whether the subject has the behavioral or mental health condition.

PARVANEH JANBAKHSHI ET AL: "Supervised Speech Representation Learning for Parkinson's Disease Classification", ARXIV.ORG, CORNELL UNIVERSITY LIBRARY, 201 OLIN LIBRARY CORNELL UNIVERSITY ITHACA, NY 14853, 1 June 2021 (2021-06-01), XP081982408, discloses auto-encoders that extract robust and discriminative speech representations for Parkinson's disease classification. To reduce the influence of speaker variabilities unrelated to pathology, it is disclosed to obtain speaker identity-invariant representations by adversarial training of an auto-encoder and a speaker identification task. To obtain a discriminative representation, it is disclosed to jointly train an auto-encoder and a pathological speech classifier.

### SUMMARY

It is an object of the invention to overcome the shortcomings in the prior art. This object of the invention is solved by the independent claims. Specific embodiments are defined in the dependent claims.

### FIGURES

Various embodiments of the present invention are described herein by way of example in conjunction with the following figures.
Figures 1 and 2 collectively illustrate a neural network-based system for discovering a voice biomarker according to various embodiments of the present invention.
Figure 3 depicts a feedforward neural network.
Figure 4 depicts a computer system according to various embodiments of the present invention.

### DESCRIPTION

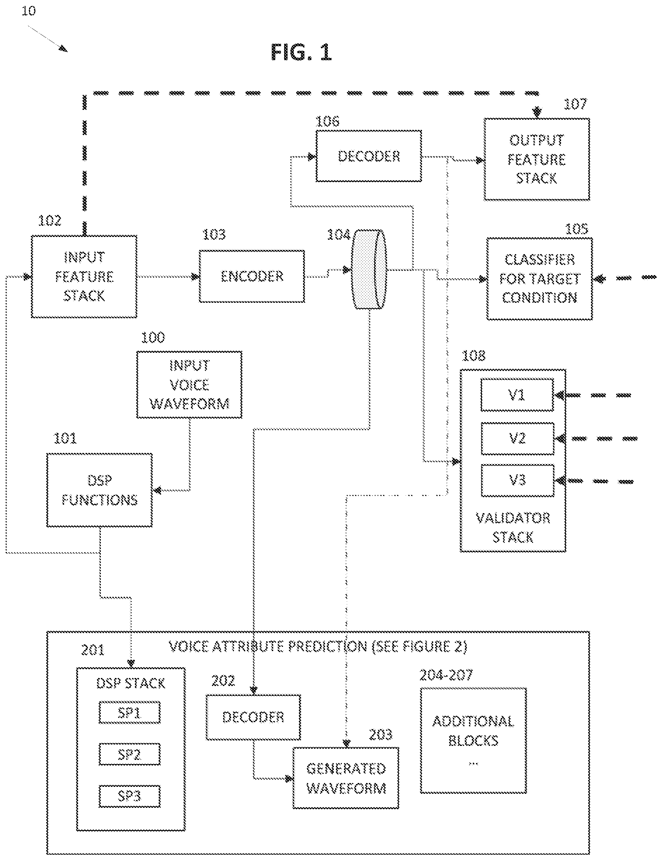

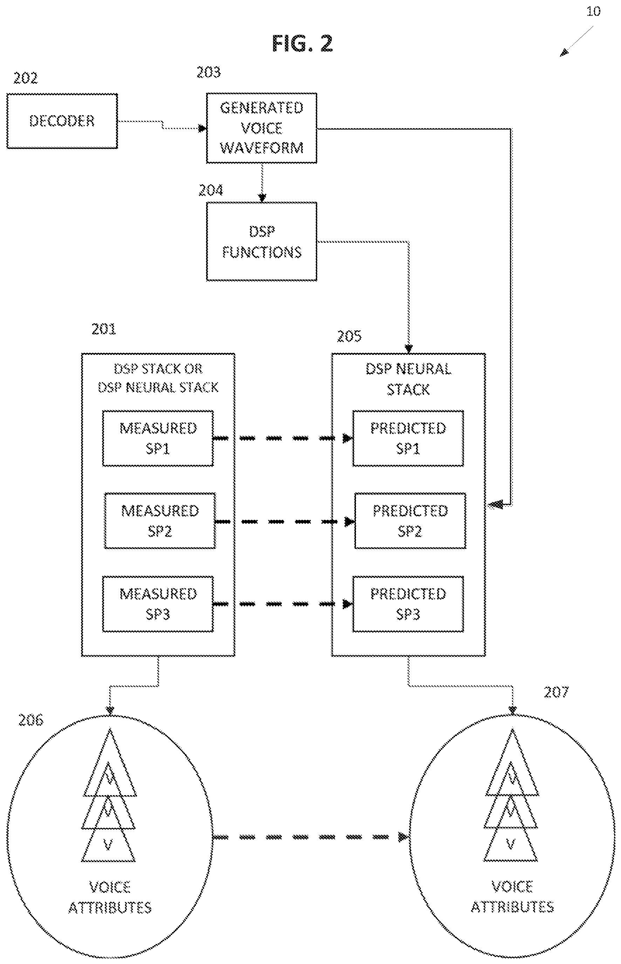

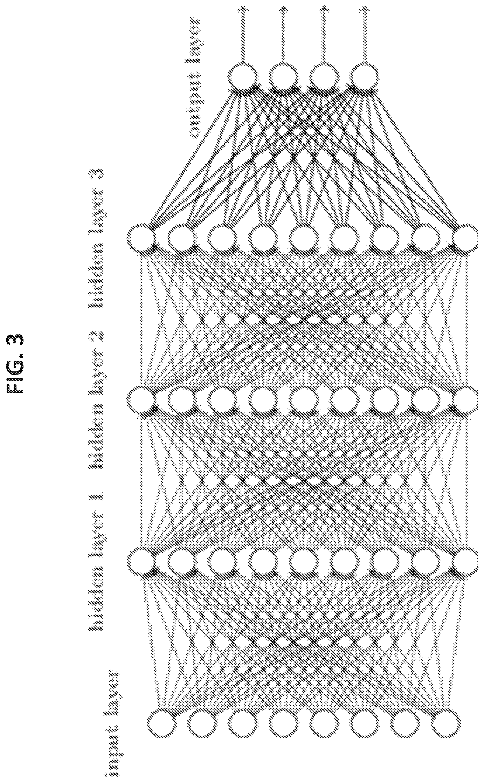

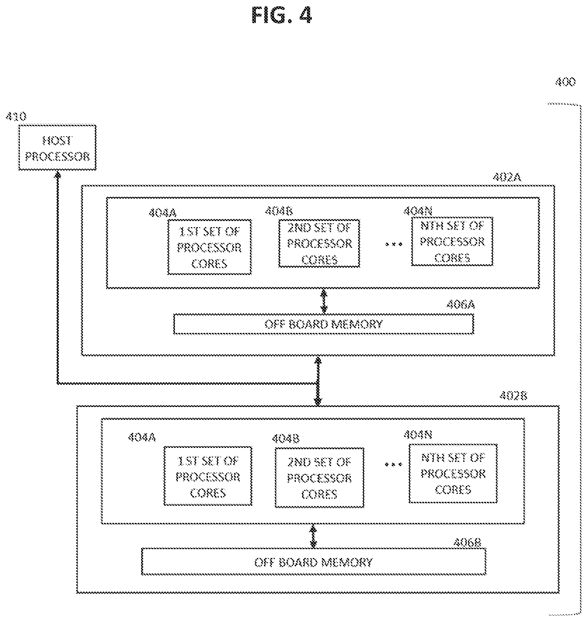

Figures 1 and 2 collectively illustrate a computer-implemented system 10 for discovery of a voice biomarker according to an illustrative embodiment of the invention. More details about certain components, e.g., components 201-207, shown in Figure 1 are shown, and described in connection with, Figure 2. Blocks 103, 105, 106, 108 in Figure 1 and blocks 201, 202, 205, 206, and 207 in Figure 2 represent various types and architectures of neural networks, such as the example neural network illustrated in Figure 3. The computations for the neural networks, including, where appropriate, forward activation and back propagation computations for training the neural network, and for other blocks of Figures 1 and 2 may be performed by a computer system, such as computer system 400 illustrated in Figure 4.

The task of the system 10 is to take an example input voice waveform and to create the biomarker 104 such that the biomarker 104 best serves to classify that example as to whether it matches a medical condition such as one of the conditions listed in Table 1 below or other medical conditions. Regardless of whether an association between voice and the given medical condition has been observed a-priori or not, the system 10 creates the engineered biomarker 104 that is most discriminative and least ambiguous for the condition. Biomarker 104 thus can be all of the following:
a) The target outcome of the system 10.
b) The final outcome of applying the process performed by the system 10.
c) The engineered biomarker embodiment created through the combined action of the system 10.
d) The discovered biomarker for the target condition processed by classifier 105.

The various neural networks in the system 10 may be trained by machine learning training algorithms such as gradient descent computed by estimating the gradient on mini batches of data examples with various refinements that are well known to those skilled in the art of training neural networks. The classifier 105 can be a neural network that is trained to match the target condition for the example input datum. In preferred embodiments of the invention, there are additional neural networks in 108 that are trained with other objectives, such as classifying for confusable medical conditions.

The neural networks represented by block 108 serve simultaneously as controls to impart the properties of discriminability for the engineered biomarker 104, to reduce the confusability of the biomarker 104, and to improve the reliability of the diagnosis that is the outcome of the classifier 105. Automated medical diagnosis based on biomarkers in voice is a challenging pattern recognition task with limited training data that is often only subjectively labeled. Furthermore, the cost of an error may be high; thus, reliability, discriminability and interpretability are top priorities.

The processing in the illustrative embodiment of Figure 1 begins with the computer system 400 recording or obtaining one or more input voice waveforms in block 100 from one or more persons, some of who have the condition for which the biomarker is to be discovered. These training voice recordings can be captured by a microphone(s) (not shown). The captured voice recordings can be digitized and stored in a computer database(s) for use by the system 10 (e.g., being used to train the system 10 through machine learning). The database(s) storing the training voice recordings could be co-located with the computer system 400 or the database(s) could be remote, in which case the computer system 400 can be in communication with the database(s) via an electronic data network, such as the Internet, a LAN, a WAN, etc.

In block 101, computer system 400 controls the selection and processing of one or more digital signal processing functions that are applied to the input voice waveform 100. For example, each of the DSP functions may compute a spectrogram, which is a representation the amplitude in each of a set of frequency channel as a function of time. The spectrogram may be computed, for example, by computing a fast Fourier transform (FFT) for each short time interval window centered around sequence of times ti.

In certain embodiments, blocks 102 in Figure 1 and block 201 in Figure 2 each compute one or more digital signal processing functions. The signal processing functions may vary, for example, in degree of resolution in time or frequency and in the bandwidth of the frequency measurements, in the type of transformations applied to the input voice signals, or in the type of representations or specific measurements derived from them. Blocks 102 and 201 may implement their respective digital signal processing functions with or without neural networks that emulate digital signal processing functions.

In the illustrative embodiment, in block 101, computer system 400 selects one or more variants of the digital signal processing functions to be used in block 102 and one or more variants of the digital signal processing to be used in block 201 of Figure 2.

Computer system 400 computes one or more of the digital signal processing functions to obtain a set of feature values 102 to provide as input to an encoder 103. This set of input features 102 may be represented, for example, as a set of one or more spectrograms, or as a matrix or higher dimensional tensor of values, depending on the implementation of the encoder 103.

In a preferred embodiment, encoder 103 is implemented with a neural network. For example, if the feature stack 102 is represented as a set of spectrograms or their images, encoder 103 may be a convolutional neural network. However, other suitable neural network architectures may be used for encoder 103 in various embodiments. The activation, training, and inference computations for neural network encoder 103 are performed on a computer system such as system 400.

Using encoder 103, computer system 400 transforms the input feature representation 102 into a latent space, yielding a latent feature representation 104. Computer system 400 can train the encoder 103 by gradient descent using back propagation backward through the latent feature representation 104.

Preferably, the encoder 103 is trained such that the latent feature representation 104 satisfies several criteria:
(1) The latent feature representation 104 is discriminative for the underlying medical condition it is engineered to encode. This criterion is achieved by back propagation training from the neural network 105.
(2) Information in the input representation 102 should not be lost. This criterion is achieved by back propagation from blocks 106 and 107, as will be explained below.
(3) The latent feature representation must be able to discriminate between the target condition and other medical conditions. This criterion is achieved by back propagation from neural networks in block 108, which are trained to recognize other medical conditions that might be confused with the target condition.

Computer system 400 can train the neural network 105 using back propagation for gradient descent based on training data examples of positive instances of the condition and on negative instances in which the condition does not exist. The arrow coming into the neural network 105 from the right indicates computer system 400 applying labeled data examples for supervised training, as do the other thick dashed-line arrows for the other neural networks in the system 10 shown in Figures 1 and 2. During training, computer system 400 can back propagate gradients from the neural network 105 to latent feature space 104 and then back to encoder 103. However, to satisfy criteria (2) and (3) above, as well as for additional reliability and interpretability, the latent feature space 104 and the encoder 103 also receive back propagation from additional subsystems, as described herein.

Computer system 400 sends the values of the latent variables 104 as input to decoder 106. The task of decoder 106 is to transform the latent variable representation 104 back into an output feature stack 107 that approximates the input feature stack 102 or any subcomponent of it that is known to be sufficient to reconstruct an approximation to the original voice signal. Computer system 400 trains decoder 106, for example, by back propagating an error loss function based on a measure of the difference between all of, or the chosen subcomponents of input feature stack 102 and output feature stack 107. That is, as indicated by the thick dashed-line arrow, input feature stack 102 is the target for training neural network decoder 106. No human labeling of the data is required in certain embodiments. Those skilled in the art know and understand this method of training neural network autoencoders. Subsystems 102, 103, 104, 106, and 107, if trained in isolation, would constitute an autoencoder.

Block 108 is a validator stack. It comprises, for example, one or more machine learning classifiers for other medical conditions that are different from the condition for which the biomarker 104 is being discovered/engineered. Three such classifiers V1, V2, and V3 are shown, but any number of classifiers may be used. The thirty (30) medical conditions listed in Table 1 are only a fraction of the medical conditions for which a neural network classifier in block 108 might be trained to classify in embodiments of the invention. The thick dashed-line arrows in Figure 1 indicate that the computer system 400 can train each of the neural network subblocks of block 108 using positive data examples and negative data examples of its respective medical condition. Computer system 400 back propagates gradients from each of the subblocks of block 108 to the latent feature representation 104 and then to encoder network 103.

In some embodiments, computer system 400 may transmit latent feature representation 104 to decoder 202 in Figure 2. In some embodiments computer system 400 may use the output of decoder 106 as a generated waveform 203 in Figure 2.

Figure 2 illustrates more details for certain components and subsystems of the system 10 shown in Figure 1. DSP stack 201 and decoder 202 can be similar in structure and operation to the DSP blocks 101-102 and decoder 106, respectively, both described above in the discussion of Figure 1. DSP stack 201 is a stack of digital single processing functions selected by computer system 400 from the functions in block 101 of Figure 1, applied by computer system 400 to input waveform 100 of Figure 1.

For each subblock of DSP stack 201, computer system 400 applies the corresponding digital signal processing function (which may include a neural network) to transform input voice waveform 100 to a feature stack like feature stack 102 of Figure 1. In some embodiments, computer system 400 may use the output of each subblock of block 201 as a target for training the corresponding neural network subblock of DSP neural stack 205.

For each subblock of DSP stack 201, computer system 400 uses the known input waveform 100 of Figure 1. For each subblock of DSP neural stack 205, computer system 400 uses as input the output of decoder 202, and/or a digital signal processing function (via DSP block 204) of generated waveform 203 and/or the generated waveform 203 itself. Being computed from the known input waveform 100, the subblock feature representations in block 201 are called "measured" signal processing features, whereas the feature representations in the subblocks of DSP neural stack 205 are called "predicted" signal processing features. DSP neural stack 205 will be discussed further below.

In some embodiments, computer system 400 uses the output of decoder 106 as the output of decoder 202. That is, for example, one decoder could be used for both the decoder 106 in Figure 1 and the decoder 202 in Figure 2.

In other embodiments, different decoders could be used. For example, computer system 400 may train a separate decoder 202 (i.e., separate from decoder 106) to generate outputs that are input to the DSP neural stack 205, and/or generate a waveform that is input to the DSP neural stack 205 after digital signal processing by DSP block 204. In these embodiments, computer system 400 uses input waveform 102 of Figure 1 as the target for decoder 202. In these embodiments, computer system 400 may use a different architecture for decoder 202 than for decoder 106 of Figure 1 and/or may use a different training procedure.

Computer system 400 transmits the output of decoder 202 and/or generated waveform 203 to DSP block 204. DSP block 204 is another stack of digital signal processing functions. In various embodiments, some, or all, of the digital signal processing functions in DSP block 204 may be the same as functions in DSP block 101 of Figure 1. In some embodiments, some, or all, of the digital signal processing functions in DSP block 204 may be different from those in DSP block 101 of Figure 1.

DSP neural stack 205 comprises a set of neural network subblocks corresponding to the subblocks of block 201. In various embodiments, computer system 400 may use one or both of two sources of back propagation for training the subblocks of DSP neural stack 205. For one source of back propagation, computer system 400 may use as a target for a subblock neural network in block 205 the measured feature representation of the corresponding subblock of block 201. For a second source of back propagation, computer system 400 may use back propagation from the computation of voice attributes systems 206 and 207, which are discussed below.

In some embodiments, computer system 400 computes estimated voice attributes by voice attribute systems 206 and 207. The voice attributes are attributes such as prosodic variations, the perceptual qualities of voice including but not restricted to assessments of having or being aphonic, biphonic, bleat/flutter, breathy, covered/muffled/darkened, creakiness, fluttery, glottalized, hoarse/raspy/harsh/grating, honky/nasal, jittery, rough/uneven/bumpy/unsteady, pressed, pulsed/vocal-fry, resonant/ringing/brightened, shimmery/crackly/buzzy, strained, strohbass, tremorous, twangy/sharp, ventricular, wobble/wavering/irregular, yawny, aesthenic; various objective measures of vocal fold dynamics such as degree of vocal fold closure, duration of adduction etc.; various objective measures of the sub-processes of voice production; and various other attributes such as those mentioned in the right-hand column of Table 1. For example, computer system 400 may train a separate neural network for each voice attribute. Thus, each voice attribute system 206, 207 may include a collection, or stack, of neural networks, with each such neural network trained to estimate respective voice attributes. In some embodiments, for each voice attribute, computer system 400 may use the same neural network for both voice attribute system 206 and the voice attribute system 207. In other embodiments, some, or all, of the attribute neural networks may be different in system 207 from the corresponding neural network in system 206.

In some embodiments, computer system 400 may use the voice attribute values computed in system 206 as targets for the voice attribute values in system 207. In some embodiments, the neural networks in system 206 and 207 are trained only from the target values in the voice attribute training data. In these embodiments, computer system 400 may pretrain the voice attribute neural networks in systems 206 and 207 and then back propagate the gradients from the system 206 target values back through the system 207 neural networks as a second source of back propagation to the stack of neural networks in system 205. In some embodiments, computer system 400 may use the system 206 target values in training the voice attribute neural networks in system 207 as well as back propagating the gradients from the system 206 target values to the neural networks in the DSP neural stack 205.

The inter-connected neural network subsystems of system 10 may be trained with a global, system-wide loss function, so that the various neural network subsystems are trained with a collective objective (in addition to local objectives that each neural network is trained with). The result of the simultaneously global and local objective training for the neural networks is the biomarker latent space 104, which can be used, once discovered as described herein, for many purposes, such as training machine learning classifiers to detect the condition corresponding to the biomarker in voice recordings. The system 10 may be trained with one or more voice recording waveforms of suitable duration.

Many of the subsystems of system 10, such as the encoder 103, decoder 106 and/or 202, classifier 105, validator stack subblocks 108, neural stack subblocks 201 and 205, and voice attribute systems 206, 207, may comprise a neural network. Figure 3 is a drawing of an example of a multi-layer feed-forward deep neural network. A neural network is a collection of nodes and directed arcs. The nodes in a neural network are often organized into layers. In a feed-forward neural network, the layers may be numbered from bottom to top, when diagramed as in Figure 3, with the input layer at the bottom and the output layer at the top conventionally. In other publications, the layers may be numbered from top to bottom or from left to right. No matter how the figure is drawn, feed forward activation computations proceed from lower numbered layers to higher number layers (i.e., from input to output), and the backpropagation computation proceeds from the highest numbered layers to the lower numbered layers (i.e., from output to input). Each directed arc in a layered feed-forward neural network goes from a source node in a lower numbered layer to a destination node in a higher numbered layer. The feed-forward neural network shown in Figure 3 has an input layer, an output layer, and three inner layers. An inner layer in a neural network is also called a "hidden" layer. Each directed arc is associated with a numerical value called its "weight." Typically, each node other than an input node is associated with a numerical value called its "bias." The weights and biases of a neural network are called "leamed" parameters. During training, the values of the learned parameters are adjusted by the computer system 400 shown in Figure 4. Other parameters that control the training process are called hyperparameters.

**Table 1: A List of Example Health Conditions and How They Tend to Affect a Person's Voice**

| Abbreviations used for parameter type: **Ac:** Acoustic, **Ar:** Articulatory, **Ev:** Evolutionary, **Md:** Medical, **Pe:** Perceptual, **Ph:** Phonatory, **Pr:** Prosodic | | |
|---|---|---|
| **No.** | **Name of syndrome** | **Effect on voice** |
| 1 | 22q11.2 deletion syndrome | Pe: Nasal |
| 2 | Anorexia Nervosa | Md: Hyperfunctional dysphonia; Ph: Significantly short phonation times; Ac: Lower fundamental frequency F0, narrowing of the voice scale |
| 3 | Anterior operculum syndrome | Md: Aphonia |
| 4 | Bardet-Biedl syndrome | Pe: Breathy, hypernasal, high pitched, poor volume control; Pr: Slow speech; Ar: Uncoordinated oral and palatal movements, misarticulations, substitutions (particularly of the first consonant of a word), and omissions of the last syllable of a word. |
| 5 | Bilateral opercular syndrome | Md: Aphonia, anarthria, palatal palsy. |
| 6 | Bogart-Bacall syndrome | Pe: Breathy, raspy, diplophonic. |
| 7 | Cerebellar motor syndrome (CMS) | Pe: Nasal; Ar: Slurred speech; Md: Ataxic dysarthria (with scanning - explosive with staccato - feature) |
| 8 | Cri-du-chat syndrome | Pe: Breathy, raspy. |
| 9 | Down syndrome | Pe: Breathy and rough (moderately), Poor voice quality, monopitch, andmonoloudness; Ac: Higher mean F0, lower harmonics-to-noise ratio; Ph: Longer voice onset times. |
| 10 | Foix-Chavany-Marie syndrome | Pe: Breathy; Ph: Short duration of phonation (max 2s). |
| 11 | Goldenhar syndrome | Pe: Breathy, harsh, high-pitched |
| 12 | KCNK9 imprinting syndrome | Md: Dysphonia; Pe: Muffled |
| 13 | Kabuki syndrome | Md: Dysarthria (with imprecise consonants, harsh voice, hypernasality, reduced rate and stress, distorted pitch), impaired Oromotor functioning; Ev: Delayed articulation and phonology |
| 14 | Mild Cognitive Impairment syndrome (MCI) | Pe: Breathy, creaky; Ac: Strong H1, weaker H2 |
| 15 | Lujan-Fryns syndrome | Pe: Breathy, raspy |
| 16 | Nager syndrome | Ph: Altered phonetic placement; Ae: Modification of hypernasality and nasal emission; Pe: Diplophonic Ac: Low intensity |
| 17 | Ortner syndrome | Pe: Hoarse, asthenic. |
| 18 | Progressive Supranuclear Palsy and Corticobasal Syndromes | Ev: Shorter speech segments, longer pauses, higher pause rate, slower speech; Ac: Reduced fundamental frequency (f0) range |
| 19 | Pulmonary parenchymal syndrome | Ac: Increased voice fremitus |
| 20 | Rett syndrome | Pe: Breathy. Ar/Ph: proto-vowel and protoconsonant alternations produced on ingressive airstream. |
| 21 | Shprintzen-Goldberg syndrome | Md: Oral hypotonia; Pe: High-pitched soft voice, slight hypernasality |
| 22 | Simpson-Golabi-Behmel syndrome | Ar: Distorted articulation; Ac: Distorted resonances; Pr: Monotonic prosody; Ev: Fluency failures |
| 23 | Sjögren's syndrome | Md: Dysphonia Pe: Diplophonic, harsh, raspy. |
| 24 | Tay syndrome | Md: Dysphonia; Pe: Hoarse, raspy, high-pitched |
| 25 | Tourette's syndrome | Md: Dysphonia |
| 26 | Wallenberg syndrome | Pe: Breathy, moderately hyponasal |
| 27 | Werner's syndrome | Pe: Hoarse, high pitch |
| 28 | Wilson-Mikity Syndrome | Pe: Biphonic |
| 29 | Syndrome of the Jugular Foramen | Md: Partial aphonia; Pe: Hoarse |
| 30 | Worster-Drought Syndrome | Md: Severe, persistent aphonia |

Once the machine learning components of the system 10 are trained, the system 10 can be used to generate the engineered voice biomarker 104 for a subject human and to determine whether the subject human has the target medical condition (or more particularly, compute a likelihood that the subject human has the target medical condition) based on the classifications of the engineered biomarker 104 by the classifiers 105, 108. The system 10 can also determine (or compute a likelihood of) whether the subject human has voice attributes associated with the target medical condition based on the DSP neural stack 205's computation of the voice attributes 207. A voice recording of sufficient duration can be captured by a microphone. The microphone could be co-located with (and/or part of) the computer system 400, or it could be remote from the computer system 400. For example, the subject human's voice recording could be captured by the microphone and then digitized, with the digitized voice recording stored in a database (such as in the cloud), where the database is in communication with the computer system 400 via an electronic data network, such as the Internet, a LAN, a WAN, etc. The microphone may include a diaphragm that is vibrated by the sound waves from the subject human's audible utterances. The vibrations of the diaphragm can be converted to an analog signal, which can be converted to digital by an analog-to-digital converter. The digital signal can be converted to a digital audio format, lossy or lossless, such as MP3, WAV, AIFF, AAC, OGG, FLAC, ALAC, WMA, etc., for storing in the database.

Referring to Figure 1, the subject human's digitized voice recording can be processed by the applicable DSP functions at block 101. The set of measurements from the DSP functions are then encoded by the encoder 103 (after having been trained) to generate the voice biomarker 104 for the subject human. The voice biomarker 104 is discriminative for the target medical condition based on how the neural network system 10 was trained, as described herein. The classifiers 105, 108 can then compute a likelihood of whether the subject human has the target medical condition, and the DSP neural stack 205 can compute the likelihood of whether the subject human has voice attributes associated with the target medical condition.

Figure 4 is a diagram of a computer system 400 that could be used to implement the embodiments described above. The illustrated computer system 400 comprises multiple processor units 402A-B that each comprises, in the illustrated embodiment, multiple (N) sets of processor cores 404A-N. The processor cores 404A-N may include one or more digital signal processors (DSPs) that perform the digital signal processing described herein, such as for blocks 101 and 204 of Figures 1 and 2, respectively. Each processor unit 402A-B may comprise on-board memory (ROM or RAM) (not shown) and off-board memory 406A-B. The on-board memory may comprise primary, volatile and/or non-volatile storage (e.g., storage directly accessible by the processor cores 404A-N). The off-board memory 406A-B may comprise secondary, non-volatile storage (e.g., storage that is not directly accessible by the processor cores 404A-N), such as ROM, HDDs, SSD, flash, etc. The processor cores 404A-N may be CPU cores, GPU cores and/or AI accelerator cores. GPU cores operate in parallel (e.g., a general-purpose GPU (GPGPU) pipeline) and, hence, can typically process data more efficiently that a collection of CPU cores, but all the cores of a GPU execute the same code at one time. AI accelerators are a class of microprocessor designed to accelerate artificial neural networks. They typically are employed as a co-processor in a device with a host CPU 410 as well. An AI accelerator typically has tens of thousands of matrix multiplier units that operate at lower precision than a CPU core, such as 8-bit precision in an AI accelerator versus 64-bit precision in a CPU core.

In various embodiments, the different processor cores 404 may train and/or implement different networks or subnetworks or components. For example, in one embodiment, the cores of the first processor unit 402A may implement the digital signal processing functions in block 101 of Figure 1 and the second processor unit 402B may implement decoder 103. As another example, the cores of the first processor unit 402A may implement the training of the neural network encoder 103 and the neural network decoder 106, the cores of the second processing unit 402B may implement the training of the neural network classifier 105, and the cores of yet another processing unit may implement the neural network classifier V1, V2, V3, ... in the validator stack 108. One or more host processors 410 may coordinate and control the processor units 402A-B.

In other embodiments, the system 400 could be implemented with one processor unit 402. In embodiments where there are multiple processor units, the processor units could be co-located or distributed. For example, the processor units 402 may be interconnected by data networks, such as a LAN, WAN, the Internet, etc., using suitable wired and/or wireless data communication links. Data may be shared between the various processing units 402 using suitable data links, such as data buses (preferably high-speed data buses) or network links (e.g., Ethernet).

The software for the various computer system 400s described herein and other computer functions described herein may be implemented in computer software using any suitable computer programming language such as .NET, C, C++, Python, and using conventional, functional, or object-oriented techniques. Programming languages for computer software and other computer-implemented instructions may be translated into machine language by a compiler or an assembler before execution and/or may be translated directly at run time by an interpreter. Examples of assembly languages include ARM, MIPS, and x86; examples of high level languages include Ada, BASIC, C, C++, C#, COBOL, Fortran, Java, Lisp, Pascal, Object Pascal, Haskell, ML; and examples of scripting languages include Bourne script, JavaScript, Python, Ruby, Lua, PHP, and Perl.

The examples presented herein are intended to illustrate potential and specific implementations of the present invention. It can be appreciated that the examples are intended primarily for purposes of illustration of the invention for those skilled in the art. No particular aspect or aspects of the examples are necessarily intended to limit the scope of the present invention. Further, it is to be understood that the figures and descriptions of the present invention have been simplified to illustrate elements that are relevant for a clear understanding of the present invention, while eliminating, for purposes of clarity, other elements. While various embodiments have been described herein, it should be apparent that various modifications, alterations, and adaptations to those embodiments may occur to persons skilled in the art with attainment of at least some of the advantages. The disclosed embodiments are therefore intended to include all such modifications, alterations, and adaptations without departing from the scope of the embodiments as set forth herein.

## Claims

1. A method comprising:
training, through machine learning, with a computer system, a neural network system to generate a biomarker that is discriminative for a target medical condition from a voice recording of a subject person;
by the computer system, the neural network system to detect whether the subject person has the target medical condition based on the voice recording of the subject person,
wherein training the neural network system comprises:
training an autoencoder with training voice recordings, wherein the autoencoder comprises an encoder and a decoder, wherein:
the encoder is trained with the training voice recordings to generate a latent feature representation from an input feature stack, wherein the input feature stack is generated from the training voice recordings; and
the decoder is trained to transform the latent feature representation to an output feature stack that approximates the input feature stack for the encoder; and
training a first classifier, with the training voice recordings, to detect the target medical condition from output from the encoder,
wherein:
the training voice recordings comprise voice recording from humans with the target medical condition and voice recording from humans without the target medical condition; and
after training of the autoencoder and the first classifier, the output of the encoder from an input, digitized voice recording of the subject person can be classified by, at least in part, the first classifier to determine whether the subject person has the target medical condition.

2. The method of claim 1, further comprising, after training the neural network system, classifying the biomarker with the first classifier to make a determination of whether the subject person has the target medical condition.

3. The method of claim 1, wherein training the encoder, the decoder, and the first classifier comprises training the encoder, the decoder, and the first classifier, with, at least, a collective objective.

4. The method of claim 3, wherein:
the encoder comprises a first neural network;
the decoder comprises a second neural network; and
the first classifier comprises a third neural network.

5. The method of claim 1, further comprising applying, by the computer system, signal processing to digitizations of the training voice recordings to compute sets of measurements from the training voice recordings.

6. The method of claim 5, wherein the sets of measurements comprise spectrograms.

7. The method of claim 2, further comprising training, by the computer system, a second classifier through machine learning, wherein the second classifier is trained to recognize another medical condition that is confusable with the target medical condition, such that after training, the output of the encoder can be classified by the second classifier to improve reliability of a classification by the first classifier.

8. The method of claim 7, wherein training the encoder, the decoder, and the first and second classifiers comprises training the encoder with, at least, a collective objective.

9. The method of claim 5, further comprising applying, by the computer system, signal processing to a digitization of the voice recording of the subject person to compute a set of measurements for the voice recording of the subject person, wherein the set of measurements is used to compute voice attributes.

10. The method of claim 9, further comprising computing, by the computer system, one or more voice attributes from the measurements computed from the voice recording.

11. The method of claim 10, further comprising training through machine learning neural networks that compute the one or more voice attributes.

12. The method of claim 1, further comprising training a second decoder to derive features from the output of the encoder for predicting voice attributes.

13. The method of claim 12, wherein training the second decoder comprises training the second decoder to reconstruct a reconstructed voice recording.

14. The method of claim 12, further comprising computing, by the computer system, a predicted voice feature from an output of the second decoder.

15. The method of claim 13, further comprising applying, by the computer system, a signal processing to the reconstructed voice recording to compute a predicted voice feature.

16. The method of one of claims 14 and 15, further comprising training through machine learning, neural networks that compute a predicted voice attribute from the predicted voice features.

17. The method of claim 1, wherein:
the encoder is part of an autoencoder that further comprises a first decoder;
the first decoder is trained to transform output from the encoder to an output feature stack that approximates an input feature stack for the encoder;
the neural network system further comprises a second decoder that reconstructs a reconstructed voice recording; and
the method further comprises:
classifying, with a first classifier, the biomarker, wherein the first classifier is trained, through machine learning, to detect the target medical condition;
applying a first signal processing to a digitization of the voice recording of the subject person to compute a set of measurements for the voice recording of the subject person;
computing one or more computed voice attributes from the measurements computed from the voice recording;
computing a predicted voice feature from output of the second decoder; and
training, through machine learning, one or more machine learning components of the neural network system using a mathematical objective obtained from the computed and predicted voice attributes, wherein the one or more machine learning components comprise one or more of the encoder, the first decoder, and the first classifier.

18. A computer system comprising: one or more processor cores; and a memory in communication with the processor cores, wherein the memory stores software that when executed by the one or more processor cores, causes the one or more processor cores to perform a method according to any one of the preceding method claims.

## Patentansprüche

1. Verfahren, umfassend:
Trainieren, durch maschinelles Lernen, mit einem Computersystem, eines neuronalen Netzwerksystems, um einen Biomarker, der für einen medizinischen Zielzustand diskriminativ ist, aus einer Sprachaufzeichnung einer Subjektperson zu erzeugen;
durch das Computersystem, des neuronalen Netzwerksystems, um basierend auf der Sprachaufzeichnung der Subjektperson zu erkennen, ob die Subjektperson den medizinischen Zielzustand aufweist,
wobei das Trainieren des neuronalen Netzwerksystems umfasst:
Trainieren eines Autocodierers mit Trainingssprachaufzeichnungen, wobei der Autocodierer einen Codierer und einen Decodierer umfasst, wobei:
der Codierer mit den Trainingssprachaufzeichnungen trainiert wird, um eine latente Merkmalsrepräsentation aus einem Eingabemerkmalsstapel zu erzeugen, wobei der Eingabemerkmalsstapel aus den Trainingssprachaufzeichnungen erzeugt wird; und
der Decodierer trainiert ist, um die latente Merkmalsrepräsentation in einen Ausgabemerkmalsstapel zu transformieren, der den Eingabemerkmalsstapel für den Codierer approximiert; und
Trainieren eines ersten Klassifikators, mit den Trainingssprachaufzeichnungen, um den medizinischen Zielzustand aus der Ausgabe des Codierers zu erkennen, wobei:
die Trainingssprachaufzeichnungen Sprachaufzeichnungen von Menschen mit dem medizinischen Zielzustand und Sprachaufzeichnungen von Menschen ohne den medizinischen Zielzustand umfassen; und
nach dem Trainieren des Autocodierers und des ersten Klassifikators, die Ausgabe des Codierers aus einer eingegebenen, digitalisierten Sprachaufzeichnung der Subjektperson mindestens teilweise durch den ersten Klassifikator klassifiziert wird, um zu bestimmen, ob die Subjektperson den medizinischen Zielzustand aufweist.

2. Verfahren nach Anspruch 1, ferner umfassend, nach dem Trainieren des neuronalen Netzwerksystems, Klassifizieren des Biomarkers mit dem ersten Klassifikator, um eine Bestimmung zu treffen, ob die Subjektperson die medizinische Zielerkrankung aufweist.

3. Verfahren nach Anspruch 1, wobei das Trainieren des Codierers, des Decodierers und des ersten Klassifikators das Trainieren des Codierers, des Decodierers und des ersten Klassifikators mit mindestens einem kollektiven Ziel umfasst.

4. Verfahren nach Anspruch 3, wobei:
der Codierer ein erstes neuronales Netzwerk umfasst;
der Decodierer ein zweites neuronales Netzwerk umfasst; und
der erste Klassifikator ein drittes neuronales Netzwerk umfasst.

5. Verfahren nach Anspruch 1, ferner umfassend ein Anwenden, durch das Computersystem, einer Signalverarbeitung auf Digitalisierungen der Trainingssprachaufzeichnungen, um Sätze von Messungen aus den Trainingssprachaufzeichnungen zu berechnen.

6. Verfahren nach Anspruch 5, wobei die Sätze von Messungen Spektrogramme umfassen.

7. Verfahren nach Anspruch 2, ferner umfassend das Trainieren, durch das Computersystem, eines zweiten Klassifikators mittels maschinellen Lernens, wobei der zweite Klassifikator derart trainiert wird, um einen anderen medizinischen Zustand zu erkennen, der mit dem medizinischen Zielzustand verwechselbar ist, dass nach dem Trainieren die Ausgabe des Codierers durch den zweiten Klassifikator klassifiziert werden kann, um eine Zuverlässigkeit einer Klassifizierung durch den ersten Klassifikator zu verbessern.

8. Verfahren nach Anspruch 7, wobei das Trainieren des Codierers, des Decodierers und des ersten und des zweiten Klassifikators das Trainieren des Codierers mit mindestens einem kollektiven Ziel umfasst.

9. Verfahren nach Anspruch 5, ferner umfassend das Anwenden, durch das Computersystem, von Signalverarbeitung auf eine Digitalisierung der Sprachaufzeichnung der Subjektperson, um einen Satz von Messungen für die Sprachaufzeichnung der Subjektperson zu berechnen, wobei der Satz von Messungen verwendet wird, um Sprachattribute zu berechnen.

10. Verfahren nach Anspruch 9, ferner umfassend das Berechnen, durch das Computersystem, eines oder mehrerer Sprachattribute aus den Messungen, die aus der Sprachaufzeichnung berechnet werden.

11. Verfahren nach Anspruch 10, ferner umfassend das Trainieren durch neuronale Netzwerke für maschinelles Lernen, die das eine oder die mehreren Sprachattribute berechnen.

12. Verfahren nach Anspruch 1, ferner umfassend das Trainieren eines zweiten Decodierers, um Merkmale aus der Ausgabe des Codierers zum Vorhersagen von Sprachattributen abzuleiten.

13. Verfahren nach Anspruch 12, wobei das Trainieren des zweiten Decodierers umfasst, den zweiten Decodierer zu trainieren, eine rekonstruierte Sprachaufzeichnung zu rekonstruieren.

14. Verfahren nach Anspruch 12, ferner umfassend das Berechnen, durch das Computersystem, eines vorhergesagten Sprachmerkmals aus einer Ausgabe des zweiten Decodierers.

15. Verfahren nach Anspruch 13, ferner umfassend das Anwenden, durch das Computersystem, einer Signalverarbeitung auf die rekonstruierte Sprachaufzeichnung, um ein vorhergesagtes Sprachmerkmal zu berechnen.

16. Verfahren nach einem der Ansprüche 14 und 15, ferner umfassend das Trainieren von neuronalen Netzwerken durch maschinelles Lernen, die ein vorhergesagtes Sprachattribut aus den vorhergesagten Sprachmerkmalen berechnen.

17. Verfahren nach Anspruch 1, wobei:
der Codierer Teil eines Autocodierers ist, der ferner einen ersten Decodierer umfasst;
der erste Decodierer trainiert ist, um eine Ausgabe des Codierers in einen Ausgabemerkmalsstapel zu transformieren, der einen Eingabemerkmalsstapel für den Codierer approximiert;
das neuronale Netzwerksystem ferner einen zweiten Decodierer umfasst, der eine rekonstruierte Sprachaufzeichnung rekonstruiert; und
das Verfahren ferner umfasst:
Klassifizieren, mit einem ersten Klassifikator, des Biomarkers, wobei der erste Klassifikator durch maschinelles Lernen trainiert ist, den medizinischen Zielzustand zu erkennen;
Anwenden einer ersten Signalverarbeitung auf eine Digitalisierung der Sprachaufzeichnung der Subjektperson, um einen Satz von Messungen für die Sprachaufzeichnung der Subjektperson zu berechnen;
Berechnen eines oder mehrerer berechneter Sprachattribute aus den Messungen, die aus der Sprachaufzeichnung berechnet werden;
Berechnen eines vorhergesagten Sprachmerkmals aus der Ausgabe des zweiten Decodierers; und
Trainieren, durch maschinelles Lernen, einer oder mehrerer Maschinenlernkomponenten des neuronalen Netzwerksystems unter Verwendung eines mathematischen Ziels, das aus den berechneten und vorhergesagten Sprachattributen erhalten wird, wobei die eine oder die mehreren Maschinenlernkomponenten einen oder mehrere von dem Codierer, dem ersten Decodierer und dem ersten Klassifikator umfassen.

18. Computersystem, umfassend: einen oder mehrere Prozessorkerne; und einen Speicher in Kommunikation mit den Prozessorkernen, wobei der Speicher Software speichert, die, wenn sie durch den einen oder die mehreren Prozessorkerne ausgeführt wird, bewirkt, dass der eine oder die mehreren Prozessorkerne ein Verfahren nach einem der vorstehenden Verfahrensansprüche durchführen.

## Revendications

1. Procédé comprenant :
l'entraînement, par apprentissage automatique, avec un système informatique, d'un système de réseau neuronal pour générer un biomarqueur qui est discriminant pour une affection médicale cible à partir d'un enregistrement vocal d'une personne sujet ;
par le système informatique, du système de réseau neuronal pour détecter si la personne sujet est atteinte de l'affection médicale cible en fonction de l'enregistrement vocal de la personne sujet,
dans lequel l'entraînement du système de réseau neuronal comprend :
l'entraînement d'un auto-encodeur avec des enregistrements vocaux d'entraînement, dans lequel l'auto-encodeur comprend un encodeur et un décodeur, dans lequel :
l'encodeur est entraîné avec les enregistrements vocaux d'entraînement pour générer une représentation de caractéristiques latentes à partir d'un empilement de caractéristiques d'entrée, dans lequel l'empilement de caractéristiques d'entrée est généré à partir des enregistrements vocaux d'entraînement ; et
le décodeur est entraîné à transformer la représentation de caractéristiques latentes en un empilement de caractéristiques de sortie qui se rapproche de l'empilement de caractéristiques d'entrée pour l'encodeur ; et
l'entraînement d'un premier classificateur, avec les enregistrements vocaux d'entraînement, pour détecter l'affection médicale cible à partir de la sortie de l'encodeur, dans lequel :
les enregistrements vocaux d'entraînement comprennent un enregistrement vocal d'humains présentant l'affection médicale cible et un enregistrement vocal d'humains ne présentant pas l'affection médicale cible ; et
après l'entraînement de l'auto-encodeur et du premier classificateur, la sortie de l'encodeur à partir d'un enregistrement vocal numérisé d'entrée de la personne sujet peut être classifiée par, au moins en partie, le premier classificateur pour déterminer si la personne sujet est atteinte de l'affection médicale cible.

2. Procédé selon la revendication 1, comprenant en outre, après l'entraînement du système de réseau neuronal, la classification du biomarqueur avec le premier classificateur pour effectuer une détermination de si la personne sujet est atteinte de l'affection médicale cible.

3. Procédé selon la revendication 1, dans lequel l'entraînement de l'encodeur, du décodeur, et du premier classificateur comprend l'entraînement de l'encodeur, du décodeur, et du premier classificateur, avec, au moins, un objectif collectif.

4. Procédé selon la revendication 3, dans lequel :
l'encodeur comprend un premier réseau neuronal ;
le décodeur comprend un deuxième réseau neuronal ; et
le premier classificateur comprend un troisième réseau neuronal.

5. Procédé selon la revendication 1, comprenant en outre l'application, par le système informatique, de traitement de signal à des numérisations des enregistrements vocaux d'entraînement pour calculer des ensembles de mesures à partir des enregistrements vocaux d'entraînement.

6. Procédé selon la revendication 5, dans lequel les ensembles de mesures comprennent des spectrogrammes.

7. Procédé selon la revendication 2, comprenant en outre l'entraînement, par le système informatique, d'un second classificateur par apprentissage automatique, dans lequel le second classificateur est entraîné à reconnaître une autre affection médicale qui peut être confondue avec l'affection médicale cible, de telle sorte qu'après l'entraînement, la sortie de l'encodeur peut être classifiée par le second classificateur pour améliorer la fiabilité d'une classification par le premier classificateur.

8. Procédé selon la revendication 7, dans lequel l'entraînement de l'encodeur, du décodeur, et des premier et second classificateurs comprend l'entraînement de l'encodeur avec, au moins, un objectif collectif.

9. Procédé selon la revendication 5, comprenant en outre l'application, par le système informatique, d'un traitement de signal à une numérisation de l'enregistrement vocal de la personne sujet pour calculer un ensemble de mesures pour l'enregistrement vocal de la personne sujet, dans lequel l'ensemble de mesures est utilisé pour calculer des attributs vocaux.

10. Procédé selon la revendication 9, comprenant en outre le calcul, par le système informatique, d'un ou plusieurs attributs vocaux à partir des mesures calculées à partir de l'enregistrement vocal.

11. Procédé selon la revendication 10, comprenant en outre l'entraînement par des réseaux neuronaux d'apprentissage automatique qui calculent le ou les attributs vocaux.

12. Procédé selon la revendication 1, comprenant en outre l'entraînement d'un second décodeur pour déduire des caractéristiques à partir de la sortie de l'encodeur pour la prédiction d'attributs vocaux.

13. Procédé selon la revendication 12, dans lequel l'entraînement du second décodeur comprend l'entraînement du second décodeur pour reconstruire un enregistrement vocal reconstruit.

14. Procédé selon la revendication 12, comprenant en outre le calcul, par le système informatique, d'une caractéristique vocale prédite à partir d'une sortie du second décodeur.

15. Procédé selon la revendication 13, comprenant en outre l'application, par le système informatique, d'un traitement de signal à l'enregistrement vocal reconstruit pour calculer une caractéristique vocale prédite.

16. Procédé selon l'une quelconque des revendications 14 et 15, comprenant en outre l'entraînement par apprentissage automatique, de réseaux neuronaux qui calculent un attribut vocal prédit à partir des caractéristiques vocales prédites.

17. Procédé selon la revendication 1, dans lequel :
l'encodeur fait partie d'un auto-encodeur qui comprend en outre un premier décodeur ;
le premier décodeur est entraîné à transformer la sortie de l'encodeur en un empilement de caractéristiques de sortie qui se rapproche d'un empilement de caractéristiques d'entrée pour l'encodeur ;
le système de réseau neuronal comprend en outre un second décodeur qui reconstruit un enregistrement vocal reconstruit ; et
le procédé comprend en outre :
la classification, avec un premier classificateur, du biomarqueur, dans lequel le premier classificateur est entraîné, par apprentissage automatique, à détecter l'affection médicale cible ;
l'application d'un premier traitement de signal à une numérisation de l'enregistrement vocal de la personne sujet pour calculer un ensemble de mesures pour l'enregistrement vocal de la personne sujet ;
le calcul d'un ou plusieurs attributs vocaux calculés à partir des mesures calculées à partir de l'enregistrement vocal ;
le calcul d'une caractéristique vocale prédite à partir de la sortie du second décodeur ; et
l'entraînement, par apprentissage automatique, d'un ou plusieurs composants d'apprentissage automatique du système de réseau neuronal à l'aide d'un objectif mathématique obtenu à partir des attributs vocaux calculés et prédits, dans lequel le ou les composants d'apprentissage automatique comprennent un ou plusieurs parmi l'encodeur, le premier décodeur, et le premier classificateur.

18. Système informatique comprenant : un ou plusieurs cœurs de processeur ; et une mémoire en communication avec les cœurs de processeur, dans lequel la mémoire stocke un logiciel qui, lorsqu'il est exécuté par le ou les cœurs de processeur, amène le ou les cœurs de processeur à effectuer un procédé selon l'une quelconque des revendications de procédé précédentes.
